# FASCICULE DE BREVET EUROPEEN

(11) **EP 3 684 487 B1**
(45) Date de publication et mention de la délivrance du brevet: **25.08.2021**
(21) Numéro de dépôt: 18762862.3
(22) Date de dépôt: 10.09.2018
(51) Int. Cl.: B01D 11/04, C07D 307/46

(54) **PROCÉDÉ DE SÉPARATION DES COMPOSÉS FURANIQUES, EN PARTICULIER LE 5- HYDROXYMETHYLFURFURAL, DU DIMETHOXYSULFOXYDE PAR DES EXTRACTIONS LIQUIDE- LIQUIDE**
VERFAHREN ZUR ABSCHEIDUNG VON FURANVERBINDUNGEN, INSBESONDERE 5-HYDROXYMETHYLFURFURAL, AUS DIMETHOXYSULFOXID DURCH FLÜSSIG-FLÜSSIG-EXTRAKTIONEN
PROCESS FOR SEPARATING FURANIC COMPOUNDS, IN PARTICULAR 5- HYDROXYMETHYLFURFURAL, FROM DIMETHOXYSULFOXIDE BY LIQUID-LIQUID EXTRACTIONS

(30) Priorité: 18.09.2017 FR 1758605
(43) Date de publication de la demande: 29.07.2020
(73) Titulaire: IFP Energies nouvelles, 92852 Rueil-Malmaison (FR)
(72) Inventeur: JACQUIN, Marc, 69002 Lyon (FR); DROZDZ, Sophie, 69126 Brindas (FR)
(74) Mandataire: IFP Energies nouvelles
(86) Numéro de dépôt international: PCT/EP2018/074276
(87) Numéro de publication internationale: WO 2019/052937

(56) Documents cités:
- WO-A1-2008/151178
- FR-A1- 2 669 635
- FR-A1- 2 669 636
- US-A1- 2004 222 153

## Description

### DOMAINE TECHNIQUE

L'invention concerne un procédé de séparation de composés furaniques, en particulier le 5-hydroxyméthylfurfural (5-HMF), le 2,5-diformylfurane (DFF), l'acide 2,5-furane dicarboxylique (FDCA) ou le 2,5-dimethyl furanoate dicarboxylique (DMFDCA), seuls ou en mélange, contenu(s) dans une charge comprenant également du diméthoxysulfoxyde (DMSO), le procédé opérant par extraction liquide-liquide.

### ART ANTERIEUR

Le 5-HMF, le DFF, le FDCA ou le DMFDCA sont des composés d'intérêt, issus de la biomasse, qui peuvent être valorisés dans de nombreux domaines notamment en pharmacie, en agrochimie ou en chimie de spécialité. En particulier, l'acide 2,5-furanedicarboxylique et le 2,5-dimethyl furanoate dicarboxylique sont des substituts de l'acide téréphtalique et respectivement du diméthyle téréphtalate en tant que monomère pour la production de fibre ou de plastique de commodité.

La production de 5-HMF par déshydratation de sucres est connue depuis de nombreuses années et a fait l'objet d'un grand nombre de travaux de recherches. Les conditions de déshydratation sont nombreuses, on peut notamment citer à titre d'exemple les méthodes suivantes :
- Le 5-HMF peut être obtenu en milieu aqueux, généralement en présence d'un catalyseur acide. Ce catalyseur acide permet de déshydrater le sucre en C6 (en particulier le fructose) en 5-HMF, mais catalyse également la réhydratation du 5-HMF en acide formique et acide lévulinique, ce qui nuit fortement au rendement.
- Le 5-HMF peut également être obtenu en milieu polaire protique, avec des solvants tels que le méthanol, l'éthanol ou l'acide acétique, et en présence d'un catalyseur acide. Dans ces conditions, le 5-HMF est obtenu en mélange avec un dérivé éther ou ester du 5-HMF en fonction du milieu réactionnel utilisé. La formation de ces produits secondaires est due à la réaction du 5-HMF avec le solvant de réaction en milieu acide.

La demande WO 2007/104514 décrit la synthèse du 5-HMF par déshydratation de sucre en utilisant le méthanol ou l'éthanol comme solvant en présence d'un catalyseur acide. Dans ce cas, la présence dudit catalyseur catalyse aussi la réaction d'éthérification du 5-HMF par l'alcool pour donner un mélange de 5-HMF et de sa forme d'éther méthylique ou éthylique suivant l'alcool utilisé comme solvant.
- Le 5-HMF peut également être produit en milieu polaire aprotique avec ou sans catalyseur acide. On peut citer plus particulièrement l'utilisation du diméthylsulfoxide (DMSO) qui, avec ou sans catalyseur acide, permet de produire du 5-HMF avec de très bons rendements, et sans les réactions indésirables listées ci-dessus.

Par ailleurs, quel que soit le milieu de synthèse (eau, méthanol, DMSO,...), des produits secondaires polymériques appelés humines sont formés lors de la production du 5-HMF (van Dam, H. E.; Kieboom, A. P. G.; van Bekkum, H. (1986) The Conversion of Fructose and Glucose in Acidic Media: Formation of Hydroxymethylfurfural. In : Starch - Stärke, vol. 38, n° 3, p. 95-101).

La synthèse du 5-HMF dans un milieu tel que le DMSO est particulièrement intéressante, car elle permet d'obtenir le 5-HMF sous sa forme alcool avec de très bons rendements. Néanmoins, les propriétés physico-chimiques du DMSO le rendent très difficilement séparable du 5-HMF par les méthodes usuelles connues de l'homme du métier.

A titre d'illustration, les méthodes de distillation du milieu réactionnel peuvent être appropriées pour éliminer l'eau présente dans le milieu réactionnel. En revanche, la distillation ne permet pas de traiter la fraction lourde contenant principalement le DMSO, le 5-HMF et les humines. En effet, le 5-HMF et les humines étant moins volatiles que le DMSO, ils se concentrent au pied de la colonne à distiller. Sous l'effet de la température et de l'augmentation de la concentration, le 5-HMF subit des réactions de dégradation, notamment de condensation et de polymérisation qui induisent une forte baisse du rendement en 5-HMF. Outre la perte de rendement en 5-HMF, on observe une forte coloration du 5-HMF de jaune à noir, ce qui peut être problématique pour son utilisation ultérieure. Pour l'ensemble de ces raisons, la distillation n'est pas donc une méthode adaptée, notamment en vue d'une industrialisation.

Par ailleurs, la cristallisation du 5-HMF dans le DMSO n'est pas envisageable pour une application industrielle. Ces deux composés forment un eutectique profond pour une composition médiane ce qui empêche la récupération de l'un ou l'autre des composés pur avec de bons rendements.

A ce jour, la seule alternative industrialisable pour l'extraction du 5-HMF d'un milieu réactionnel contenant du DMSO est l'extraction liquide-liquide, suivie d'une cristallisation de l'extrait, telle que décrite dans le brevet FR 2669635.

Selon ce brevet, dans une première étape, de l'eau est ajoutée au milieu réactionnel afin de pouvoir réaliser une extraction liquide-liquide avec un solvant non miscible à l'eau, tel que le dichlorométhane ou bien le diéthyléther. Cette technique de séparation est efficace d'un point de vue du rendement (91-98%) [comme le montrent les valeurs c9 dans les exemples 1 à 5 de ce brevet], mais l'extrait récupéré est impur. En particulier, l'extrait contient une quantité non négligeable de DMSO [valeurs c10 dans les exemples 1 à 5].

Ainsi, la pureté du 5-HMF récupéré dans l'extrait (hors solvant d'extraction) est inférieure à 90-93% dans les exemples 1 à 5 du brevet FR-2669635: la pureté étant définie comme le rapport quantité de 5-HMF / quantité de DMSO + quantité de 5-HMF soit le rapport c8/(c8+c10)*100.

Dans une seconde étape, une partie du solvant d'extraction (dichlorométhane ou diéthyléther) est évaporée de manière à produire une solution plus concentrée en 5-HMF (et en DMSO conjointement extrait avec le 5-HMF) dans le solvant d'extraction ; puis cette solution plus concentrée est refroidie dans le but de cristalliser le 5-HMF qui est récupéré par filtration. Le rendement de cristallisation est typiquement de l'ordre de 69-72% [valeurs e5 des exemples]. Cette opération est donc répétée sur le filtrat dans le but d'obtenir un rendement global de cristallisation de l'ordre 90-94% [valeurs e7 des exemples 1 à 5].

Le procédé proposé dans ce brevet FR 2669635 permet donc d'obtenir un bon rendement global d'extraction du 5-HMF en réalisant une extraction liquide-liquide, suivie d'une cristallisation de l'extrait.

Néanmoins, nous avons constaté que la présence de DMSO (conjointement extrait avec le 5-HMF) dans l'étape de cristallisation est préjudiciable.

En effet, la présence de DMSO augmente fortement la limite de solubilité du 5-HMF, et induit un retard à la nucléation et croissance des cristaux de 5-HMF. L'augmentation de la limite de solubilité a un impact négatif sur les coûts opératoires du procédé, car pour obtenir un même rendement de cristallisation en présence de DMSO, il est nécessaire de refroidir davantage le milieu. Par ailleurs, le retard à la nucléation et à la croissance des cristaux de 5-HMF a un impact négatif sur les coûts d'investissement du procédé, car pour obtenir un même rendement de cristallisation en présence de DMSO, il est nécessaire de générer davantage de temps de séjour, et donc d'utiliser des cristallisoirs plus volumineux.

Par ailleurs, le 2,5-diformylfurane (DFF) et l'acide 2,5-furane dicarboxylique (FDCA) peuvent être obtenus par oxydation du 5-HMF dans le DMSO. Par ailleurs, le 2,5-dimethyl furanoate dicarboxylique (DMFDCA) peut être obtenu par estérification de l'acide 2,5-furane dicarboxylique (FDCA), toujours dans le DMSO en présence de méthanol. Ces composés ont des propriétés physico-chimiques relativement proches du 5-HMF, et la problématique énoncée ci-dessus peut-être généralisée du 5-HMF à ces autres composés furaniques.

La demanderesse propose une amélioration du procédé décrit dans le brevet FR 2669635. Cette amélioration est basée sur la modification de l'étape d'extraction, notamment en ajoutant une étape de contre-extraction à l'eau.

On améliore ainsi la pureté du 5-HMF extrait, pour une même quantité d'eau ajoutée. On peut obtenir une pureté de 95% ou plus, voire même d'au moins 98%. En particulier, cette contre-extraction réduit la quantité de DMSO contenu dans l'extrait.

L'étape de cristallisation du 5-HMF dans l'extrait purifié peut être réalisée dans des conditions bien plus favorables. En particulier, la cristallisation du 5-HMF dans l'extrait purifié peut être opérée à des températures plus élevées (limitant ainsi la consommation énergétique) et des volumes de cristallisoirs plus faibles (limitant ainsi les coûts d'investissement).

La cristallisation de cet extrait purifié (i.e. dépourvu de DMSO) permet d'obtenir un meilleur rendement de cristallisation du 5-HMF toutes choses égales par ailleurs, et donc d'obtenir un filtrat contenant moins de 5-HMF. Le recyclage de ce filtrat contenant moins 5-HMF comme solvant d'extraction, permet alors d'obtenir un meilleur rendement d'extraction dans un procédé industriel fonctionnant en continu. L'ajout de cette étape de contre-extraction à l'eau permet donc un fonctionnement amélioré du procédé décrit dans le brevet FR 2669635, d'extraction du 5-HMF et cristallisation du 5-HMF dans le solvant d'extraction.

### OBJET DE L'INVENTION

Un objet de la présente invention est donc de fournir un procédé de séparation de composés furaniques et plus particulièrement du 5-HMF, d'une charge comprenant au moins du DMSO, par extraction liquide-liquide.

Un autre objet de l'invention est de fournir un procédé de séparation de composés furaniques et plus particulièrement du 5-HMF cristallisé dans des conditions douces.

### DESCRIPTION SOMMAIRE DE L'INVENTION

L'invention concerne un procédé de séparation du ou des composés furaniques contenus dans une charge comprenant également du diméthoxysulfoxyde (DMSO), ledit procédé comprenant successivement les étapes suivantes :
a) une étape a) dans laquelle ladite charge est mise en contact avec le raffinat intermédiaire aqueux enrichi en DMSO produit à l'étape c) et le mélange est éventuellement filtré,
b) une étape b) d'extraction dudit mélange produit à l'étape a) par un solvant organique choisi parmi les solvants chlorés, les éthers, les cétones et les aromatiques, produisant un raffinat aqueux enrichi en DMSO et un extrait organique intermédiaire enrichi en composé(s) furanique(s),
c) une étape c) de contre-extraction dudit extrait organique intermédiaire enrichi en composé(s) furanique(s) produit à l'étape b) par de l'eau ajoutée, produisant un raffinat intermédiaire aqueux enrichi en DMSO et un extrait organique purifié, et ledit raffinat intermédiaire aqueux est introduit en partie ou en totalité dans l'étape a).

La pureté du composé furanique (en particulier du 5-HMF) dans l'extrait organique purifié produit à l'étape c) est d'au moins 93%, et généralement d'au moins 95%, calculée comme étant le rapport entre la quantité de composés furaniques / quantité de composés furaniques + quantité de DMSO dans cet extrait organique purifié.

De préférence, le procédé comprend en outre une étape d) de cristallisation du (des) composé(s) furanique(s) contenu(s) dans ledit extrait purifié produit à l'étape c), suivie d'une filtration, et il est obtenu ledit composé solide d'une part et un filtrat riche en solvant d'autre part. Par riche en solvant, on entend plus de 80%pds de solvant, de préférence plus de 90%pds de solvant. Le filtrat issu de l'étape d) est alors astucieusement recyclé, en partie ou en totalité, vers l'étape b) à titre de solvant organique pour réaliser l'étape d'extraction. Il peut alors être réalisé une nouvelle extraction dudit composé furanique (et en particulier du 5-HMF).

Dans un mode préféré de l'invention, le raffinat aqueux enrichi en DMSO produit à l'étape b) est distillé, de préférence sous vide. Il est obtenu un résidu riche en DMSO d'une part et un distillat riche en eau d'autre part. Par riche, on étend ici plus de 95% pds, de préférence plus de 98%pds.

Une partie ou la totalité du distillat riche en eau peut astucieusement être recyclé à l'étape c) à titre d'eau ajoutée pour réaliser l'étape de contre-extraction. Le résidu riche en DMSO peut astucieusement être réutilisé dans le procédé amont de synthèse pour produire davantage de composé furanique (par exemple du 5-HMF).

Généralement, la quantité d'eau dans le mélange produit à l'étape a) est de 10% à 90% pds. Cette quantité d'eau est dépendante du solvant organique introduit à l'étape b).

Le solvant de l'étape b) est choisi de préférence dans le groupe formé par les solvants chlorés C1-C10, les éthers C2-C10, les cétones C2-C10, les composés aromatiques C4-C10.

Généralement, les étapes b) et c) ont lieu à une température de 0 à 60°C, de préférence de 10 à 30°C, de préférence à température ambiante.

De préférence, l'eau de contre-extraction ajoutée à l'étape c) contient moins de 1%pds de DMSO, de préférence moins de 0,1%pds de DMSO.

Le composé furanique est de préférence choisi dans le groupe formé par le 5-hydroxyméthylfurfural (5-HMF), le 2,5-diformylfurane (DFF), l'acide 2,5-furanedicarboxylique (FDCA) ou le 2,5-dimethyl furanoate dicarboxylique(DMFDCA), seuls ou en mélange.

L'invention concerne particulièrement un procédé dans lequel le composé furanique est le 5-hydroxyméthylfurfural (5-HMF).

Ainsi, l'invention porte en particulier sur un procédé d'extraction du 5-HMF par extraction liquide-liquide d'une charge comprenant du 5-HMF et du DMSO, ledit procédé comprenant successivement les étapes:
a) une étape a) dans laquelle ladite charge est mise en contact avec le raffinat aqueux intermédiaire enrichi en DMSO produit à l'étape c), et le mélange est éventuellement filtré ;
b) une étape b) d'extraction du mélange produit à l'étape a) par un solvant organique choisi parmi les solvants chlorés, les éthers, les cétones et les composés aromatiques, produisant un raffinat aqueux enrichi en DMSO, et un extrait organique intermédiaire enrichi en 5-HMF; optionnellement ledit raffinat aqueux est distillé et il est obtenu un résidu riche en DMSO d'une part et un distillat riche en eau d'autre part,
c) une étape c) de contre-extraction de l'extrait organique intermédiaire enrichi en 5-HMF produit à l'étape b) par de l'eau ajoutée, produisant un raffinat aqueux intermédiaire enrichi en DMSO à au moins 60%pds d'eau et un extrait organique purifié, et ledit raffinat aqueux intermédiaire est recyclé, en partie ou en totalité, vers l'étape a).
d) optionnellement, une étape d) de cristallisation du 5-HMF contenu dans l'extrait organique purifié produit à l'étape c), suivie d'une filtration, et il est obtenu le 5-HMF solide et un filtrat riche en solvant.

### DESCRIPTION DETAILLEE DE L'INVENTION

Elle sera faite le plus souvent en rapport avec 5-HMF mais elle peut être étendue aux autres composés furaniques mentionnés.

### La charge entrant à l'étape a)

Conformément à la présente invention, la charge du procédé de séparation contient au moins un composé furanique et du DMSO. Parmi les composés furaniques, on peut citer le 5-hydroxyméthylfurfural (5-HMF), le 2,5-diformyl furane (DFF), l'acide 2,5-furane dicarboxylique (FDCA) et le 2,5- dimethyl furanoate dicarboxylique (DMFDCA), seuls ou en mélange.

L'invention s'applique en particulier à une charge contenant du 5-HMF.

Elle est issue des procédés de fabrication du 5-HMF par déshydratation de sucres dans un milieu réactionnel comprenant du DMSO, ces procédés sont bien connus de l'homme du métier.

Par sucre on entend le glucose ou le fructose, seuls ou en mélange, le saccharose, mais aussi les oligosaccharides tels que le cellobiose, la cellulose ou bien encore l'inuline. Par sucre, on entend donc généralement un sucre contenant 6 atomes de carbone (hexoses), mais ceci n'exclut pas la présence dans la charge de sucres contenant 5 atomes de carbone (pentoses).

Le DMSO représente généralement plus de 40% poids de la charge du procédé de séparation, voire plus de 60% poids de la charge du procédé de séparation.

Le ou les composés furaniques représentent plus de 1% poids de la charge du procédé de séparation, de préférence plus de 10% poids, de préférence plus de 20% poids.

Par ailleurs, cette charge peut contenir de l'eau avant même son mélange avec le raffinat aqueux intermédiaire dans l'étape a). Cette eau peut avoir été formée par la réaction de déshydratation du sucre en 5-HMF (3 moles d'eau par mole de 5-HMF), ou encore par la réaction d'estérification du FDCA en DMFDCA (2moles d'eau par mole de FDCA). De même, cette eau peut également avoir été introduite avec le sucre, dans le cas où pour des raisons pratiques, un sirop de sucre à environ 70% poids dans l'eau avait été utilisé.

Ainsi, la charge peut contenir de l'eau, dans une proportion généralement comprise entre 0,1 et 30 % poids, de préférence entre 0,1 et 15% en poids.

### Le solvant organique d'extraction entrant à l'étape b)

Conformément à l'invention, le solvant organique d'extraction introduit à l'étape b) est choisi parmi les solvants non miscibles avec l'eau, de manière à former deux phases liquides à l'étape c) de contre-extraction, mais aussi à l'étape b) d'extraction en présence d'une forte proportion de DMSO dans le raffinat. Cette propriété est fortement dépendante de la proportion relative des débits de charge, d'eau de contre-extraction et de solvant d'extraction mis en œuvre dans le procédé.

De manière non limitative, le solvant est choisi parmi la famille des solvants chlorés, des éthers, des cétones et des composés aromatiques. De préférence ce sont des solvants chlorés C1-C10, des éthers C2-C10, des cétones C2-C10, des composés aromatiques C4-C10. De manière préférée, le solvant est choisi parmi le dichlorométhane, le diethyléther, le diisopropyléther, la méthyléthylcétone, la methylisopropylcétone, le thiophène, l'anisole et le toluène. De manière très préférée, le solvant est le dichlorométhane.

De préférence, le solvant est pur (du moins de pureté commerciale).

A noter que dans le mode préféré de l'invention où le filtrat riche en solvant organique obtenu à l'étape d) est recyclé à l'étape b), ce dernier peut contenir une quantité résiduelle de composé furanique et de DMSO.

La quantité résiduelle de DMSO sera d'autant plus faible que la contre-extraction aura été réalisée de manière efficace à l'étape c).

La quantité résiduelle de composé furanique sera d'autant plus faible que la cristallisation aura été réalisée de manière efficace à l'étape d).

### L'eau de contre-extraction ajoutée à l'étape c)

On entend par eau de contre-extraction ajoutée à l'étape c) un flux qui contient plus de 95% pds d'eau, de préférence plus de 98% pds d'eau.

A noter que dans le mode préféré de l'invention où le raffinat aqueux enrichi en DMSO produit à l'étape b) est distillé, et que le distillat riche en eau ainsi obtenu est utilisé pour alimenter l'étape c), ce dernier peut encore contenir une quantité résiduelle de DMSO.

La quantité résiduelle de DMSO sera d'autant plus faible que la distillation aura été réalisée de manière efficace à l'étape b).

L'efficacité du contre-lavage sera d'autant plus forte que la quantité de DMSO présente dans l'eau de contre-extraction ajoutée sera faible. De manière préférée, l'eau de contre-extraction ajoutée contient donc moins de 1% pds de DMSO, de préférence moins de 0,1 % pds de DMSO.

### L'étape a) de mélange

Selon l'invention, ladite charge contenant au moins un composé furanique et du DMSO, est mélangée avec le raffinat intermédiaire aqueux enrichi en DMSO issu de l'étape c) de contre-extraction, raffinat intermédiaire aqueux qui est introduit en partie ou en totalité dans l'étape a). Ce raffinat intermédiaire aqueux contient plus de 60% pds d'eau, de manière préférée plus de 80% pds d'eau.

Le mélange résultant contient 10% à 90% pds d'eau, de préférence 20-80% pds d'eau.

En augmentant la teneur en eau, une partie des éventuels sous-produits « humines » précipitent. On appelle « humines » l'ensemble des composés polymériques indésirables formés lors de la synthèse du 5-HMF. Les humines peuvent représenter jusqu'à 30% pds de la charge, et souvent sont de l'ordre de 20%pds. Elles peuvent être quantifiées de différentes manières bien connues de l'homme du métier, comme par exemple par chromatographie d'exclusion stérique. Une opération optionnelle de filtration permet ainsi d'éliminer les « humines » qui ont précipité. Le filtrat obtenu est alors envoyé vers l'étape b) d'extraction liquide-liquide. Lorsque la quantité d'humines dans la charge est faible (par exemple 1% pds ou moins), on peut se passer de l'étape a) de filtration.

L'étape a) a lieu généralement à une température de 0 à 60°C, de préférence de 10 à 30°C et généralement à température ambiante.

### L'étape b) d'extraction

L'extraction liquide-liquide réalisée à l'étape b) est une extraction du mélange obtenu à l'étape a) à contre-courant du solvant organique. Cette technique est bien connue de l'homme du métier. L'extraction peut être réalisée par exemple dans une batterie de mélangeur-décanteur, dans une colonne remplie de garnissage vrac ou structuré, dans une colonne pulsée, ou bien encore dans une colonne agitée.

L'extraction est réalisée généralement à une température comprise entre 0 et 60°C, de préférence entre 10 et 30°C, soit le plus souvent à température ambiante.

La proportion (pds/pds) de solvant est de préférence de 0.2 à 5 par rapport au mélange introduit, le rapport peut être supérieur (par ex jusqu'à 70) dans des colonnes agitées.

On récupère d'une part un flux appauvri en composé furanique, appelé raffinat aqueux, qui contient une grande partie du DMSO contenu initialement dans la charge, et d'autre part un flux enrichi composé furanique, appelé extrait organique intermédiaire, qui contient une grande partie du ou des composés furaniques contenus initialement dans la charge. Cet extrait intermédiaire contient aussi un peu de DMSO.

L'extrait organique intermédiaire enrichi en 5-HMF est dirigé vers l'étape c) de contre-extraction.

Le raffinat aqueux peut subir différents traitements afin de séparer l'eau du DMSO qui le constituent majoritairement. Ainsi, dans un mode préféré de l'invention, le raffinat aqueux est distillé sous vide pour récupérer un résidu riche en DMSO d'une part et d'autre un distillat riche en eau.

Ce résidu riche en DMSO peut être avantageusement envoyé dans une unité de synthèse du 5-HMF (par exemple par déshydratation de sucres).

Le distillat riche en eau peut, en totalité ou en partie, être avantageusement recyclé à l'étape c) à titre d'eau ajoutée.

### L'étape c)

Selon l'invention, l'extrait organique intermédiaire enrichi en 5-HMF obtenu à l'étape b) est soumis à une étape de contre-extraction par de l'eau ajoutée.

L'introduction d'eau est réalisée de façon à mettre en œuvre l'extraction, selon les connaissances générales de l'homme du métier. Il est bien certain que l'ajout d'eau est le plus faible possible de façon à réduire les coûts, mais suffisant pour garantir une pureté du composé furanique d'au moins 93%, de préférence d'au moins 95%. La quantité d'eau ajoutée est telle que la quantité d'eau dans le mélange de l'étape a) est comprise entre 10-90% pds de la charge, souvent entre 20-80%.

L'extraction liquide-liquide réalisée à l'étape c) est une extraction de l'extrait organique intermédiaire obtenu à l'étape b) à contre-courant de l'eau ajoutée. Cette technique est bien connue de l'homme du métier. L'extraction peut être réalisée par exemple dans une batterie de mélangeur-décanteur, dans une colonne remplie de garnissage vrac ou structuré, dans une colonne pulsée, ou bien encore dans une colonne agitée.

L'extraction est réalisée généralement à une température comprise entre 0 et 60°C, de préférence entre 10 et 30°C, soit le plus souvent à température ambiante.

La proportion (pds/pds) de solvant est de préférence de 0.2 à 5 par rapport au mélange introduit, le rapport peut être supérieur (par ex jusqu'à 70) dans des colonnes agitées.

On récupère un flux aqueux enrichi en DMSO, appelé raffinat aqueux intermédiaire, contenant généralement au moins 60% pds d'eau, de préférence au moins 80% pds d'eau, et un extrait organique purifié. Ce raffinat aqueux intermédiaire est de préférence introduit, en partie ou de préférence en totalité, dans l'étape a).

Le composé furanique ou plus particulièrement le 5-HMF contenu dans l'extrait obtenu présente une pureté supérieure à 93% pds, et généralement de 95% ou plus, voire même d'au moins 98% (quantité de 5-HMF/quantité de 5-HMF+quantité de DMSO).

Parallèlement, on a donc réduit la quantité de DMSO contenu dans l'extrait organique (d'au plus 7% pds, généralement d'au plus 5% ou d'au plus 2% pds hors solvant d'extraction). Plus la teneur en DMSO est faible, plus l'étape de cristallisation du composé furanique (ou plus particulièrement du 5-HMF) contenu dans l'extrait sera réalisée dans des conditions favorables. On améliore ainsi le rendement (et éventuellement la pureté) en composé furanique (ou plus particulièrement en 5-HMF) cristallisé.

### L'étape d) de cristallisation

De façon avantageuse, l'extrait organique purifié obtenu à l'étape c) subit une étape de cristallisation et une étape de filtration. Il est produit des cristaux de composé furanique (ou plus particulièrement de 5-HMF) et un filtrat riche en solvant.

La cristallisation peut être réalisée par tous les modes connus de l'homme du métier, comme par exemple par abaissement de la température, augmentation de la concentration en composé à cristalliser, augmentation de la concentration en composé à cristalliser avec abaissement de la température, ou bien encore par l'ajout d'un tiers composé qui est un mauvais solvant du composé à cristalliser.

Les conditions sont celles connues de l'homme du métier. Dans le cas du 5-HMF, la température est inférieure ou égale à 30°C, et généralement entre -100°C et 0°C, de préférence entre -50°C et 0°C et de manière encore plus préférée entre -40°C et -10°C. Dans le cas des autres dérivés furaniques que l'on peut produire dans le DMSO à partir du 5-HMF, dont la température de fusion est supérieure à celle du 5-HMF, la température est inférieure ou égale à 150°C, et généralement entre -100°C et 30°C, de préférence entre -50°C et 30°C.

Selon l'invention, le filtrat riche en solvant est avantageusement recyclé à l'étape b) pour réaliser une nouvelle extraction du 5-HMF ou autres composés furaniques.

Le filtrat est de préférence recyclé, en partie ou en totalité (après une éventuelle purge), vers l'étape b) d'extraction liquide-liquide.

Selon les méthodes connues de l'homme du métier, l'étape d) est réalisée en une ou plusieurs passes, les cristaux sont lavés et séchés.

### Description des figures

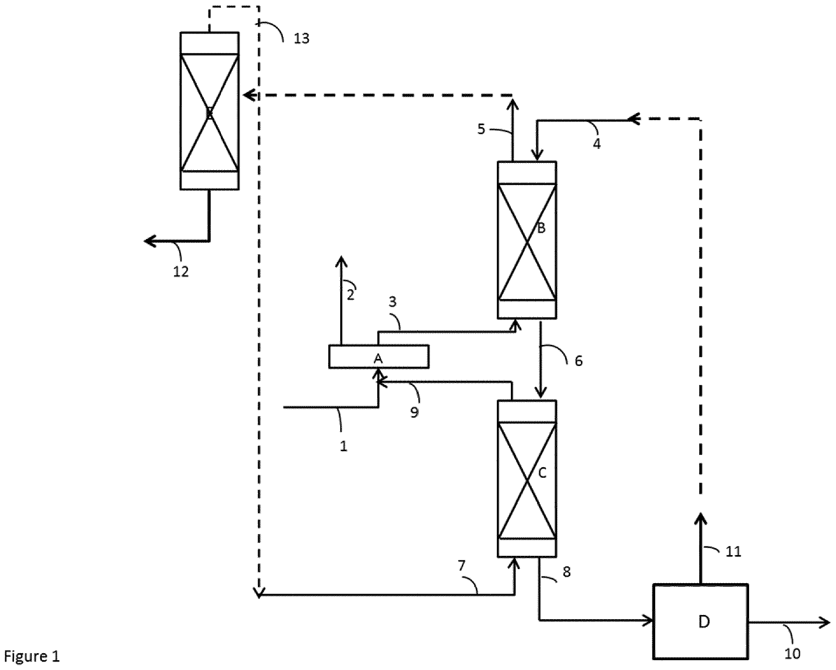

Selon la figure 1, qui présente un mode de réalisation préféré, la charge 1 contenant au moins du 5-HMF ou autres composés furaniques et du DMSO est mélangée selon l'étape a) dans la section de mélange A avec le raffinat aqueux intermédiaire 9 issu de l'étape c) de contre-extraction.

Selon la figure 1, les « humines » précipités sont filtrés dans la zone de filtration A et sont évacués par la conduite 2. Cette zone peut ne pas exister ou être by-passée, notamment dans le cas où la quantité d'humides dans la charge est faible.

Le mélange obtenu 3 (filtré ou non) est envoyé vers un extracteur liquide-liquide B pour réaliser l'étape b) d'extraction du composé furanique. Le mélange 3 circule à contre-courant du solvant d'extraction 4. Il est produit d'une part un raffinat aqueux 5 appauvri en composé furanique et d'autre part un extrait organique intermédiaire 6 enrichi en composé furanique.

L'extrait organique intermédiaire 6 enrichi en 5-HMF est dirigé vers un deuxième extracteur liquide-liquide C pour réaliser l'étape c) de contre-extraction à l'eau. Il circule à contre-courant de l'eau 7 de contre-lavage ajoutée. Il est produit d'une part un raffinat aqueux intermédiaire 9 riche enrichi en DMSO et d'autre part un extrait organique purifié 8.

L'extrait organique purifié 8 subit une étape de cristallisation d), par exemple par refroidissement, dans un cristallisoir D. Les cristaux de composés furaniques en suspension dans le solvant sont ensuite récupérés par filtration en séchés. Le composé furanique est récupéré sous forme solide avec une haute pureté (référence 10). Le filtrat 11 riche en solvant est recyclé à titre de solvant d'extraction vers l'extracteur B pour réaliser une nouvelle étape b) d'extraction.

Le raffinat aqueux 5 subit par exemple une distillation dans la colonne à distiller E, de manière à produire un résidu riche en DMSO 12, et un distillat riche en eau 13. Le distillat riche en eau 13 est recyclé vers l'extracteur C à titre de d'eau ajoutée pour réaliser une nouvelle étape c) de contre-extraction.

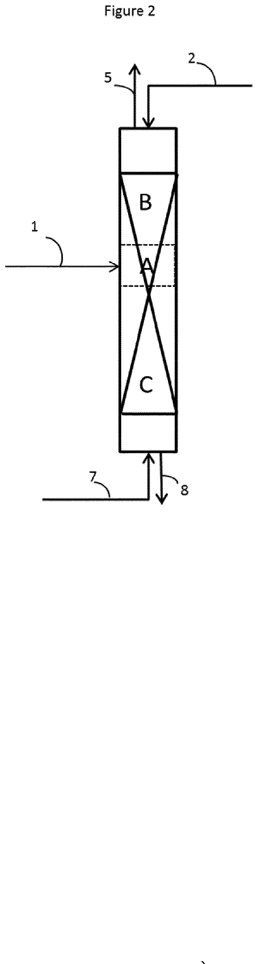

Les figures illustrent un mode préféré de l'invention sans la limiter. Ainsi, dans un mode de réalisation de l'invention représenté figure 2, les étapes b) d'extraction et c) de contre-extraction peuvent être réalisées dans une seule et même opération unitaire notamment lorsque la concentration en humides est faible (1% pds ou moins). Ainsi, une même colonne d'extraction liquide-liquide est utilisée pour réaliser les étapes b) et c). La charge 1 entre dans cette colonne en un point intermédiaire situé entre le point d'injection du solvant et le point d'injection de l'eau de contre-lavage. Ce point d'injection de la charge constitue la zone A. En supposant que le solvant d'extraction soit plus dense que l'eau, la partie supérieure audit point d'injection de la charge constitue la zone B d'extraction et la partie inférieure audit point d'injection constitue la zone C de contre-extraction.

La zone B d'extraction opère selon l'étape b). L'extrait organique intermédiaire issu de la zone B d'extraction (qui correspond à la référence 6 de la figure 1) descend par gravité dans la zone C de contre-extraction, en passant ici par la zone de mélange A.

La zone C de contre-extraction opère selon l'étape c). Le raffinat aqueux intermédiaire issu de la zone C de contre-extraction (qui correspond à la référence 9 de la figure 1.) monte par gravité dans la zone d'extraction B, en passant par la zone de mélange A où il se retrouve au contact de la charge 1. L'extrait purifié 8 qui sort de la zone C de contre-extraction peut avantageusement subir une étape de cristallisation d).

Ainsi, dans le mode de réalisation de la figure 1, les étapes a), b) et c) sont réalisées dans des zones différentes séparées. Par contre, dans le mode de réalisation de la figure 2, les étapes a), b) et c) sont réalisées dans des zones différentes non séparées, ce mode convient bien lorsque la quantité d'humines est de 1% poids ou moins (mesuré par chromatographie d'exclusion stérique).

### EXEMPLES

Les exemples ci-dessous illustrent l'invention sans en limiter la portée.

### Exemple 1 : procédé selon l'invention

Afin de montrer les avantages de la présence d'une étape de contre-extraction, nous présentons ici les résultats de simulation de l'étape c) du procédé selon l'invention opéré selon la figure 1.

Les conditions opératoires sont pour l'étape a) : un rapport pondéral DMSO/5-HMF dans la charge de 55/45, et il n'y a pas d'eau dans la charge. Le débit de charge est de 100 kg/h. Le débit d'eau provenant de l'étape c) est le même dans tous les cas simulés.

Les conditions opératoires de l'étape b) : le solvant est du DCM pur (pas de DMSO ni de 5-HMF). Le débit de solvant est de 185 kg/h. Le nombre d'étages théoriques de la section d'extraction est constant et fixé à 4. L'étape est opérée à une température de 20°C.

Les conditions opératoires de l'étape c) : de l'eau pure est utilisée, avec un débit de 100 kg/h. La température est de 20°C. Nous faisons varier le nombre d'étages théoriques de la section de contre-extraction (appelé NET contre-lavage dans le tableau 1). Plus ce dernier est faible, et plus l'on tend vers le procédé décrit dans le brevet FR2669635 (aucun étage de contre-extraction).

L'outil de simulation utilisé établit un bilan matière sur chaque constituant, étage par étage, en respectant un partage des constituants entre les deux phases au sein de chaque étage. Ce partage des constituants a été préalablement mesuré au laboratoire, pour des compositions caractéristiques de la section d'extraction (phase aqueuse riche en DMSO et phase solvant pauvre en 5-HMF) et de la section de contre-extraction (phase aqueuse pauvre en DMSO et phase solvant riche en 5-HMF).

**Tableau 1**

| NET contre-lavage | Rendement d'extraction 5-HMF (%) | Rendement d'extraction DMSO (%) |
|---|---|---|
| 1 | 99,1 | 8,7 |
| 2 | 99,0 | 2,6 |
| 3 | 99,0 | 0,8 |
| 4 | 99,0 | 0,2 |
| 5 | 99,0 | 0,1 |
| 6 | 99,0 | 0 |

Le rendement d'extraction est défini comme la quantité de 5-HMF (ou respectivement de DMSO) récupéré dans l'extrait produit à l'étape c), divisé par la quantité de 5-HMF (ou respectivement de DMSO) introduit dans la charge.

Le tableau ci-dessus montre que les conditions de l'étape c) sont ajustées en fonction de la quantité de DMSO acceptable dans l'extrait envoyé en cristallisation.

### Exemple 2 : Couplage du procédé selon l'invention avant NET=1 et du procédé de cristallisation à -18°C

Dans cet exemple, on illustre l'effet synergique qui existe entre le procédé d'extraction liquide-liquide selon l'invention, et le procédé de cristallisation.

Reprenons les résultats de l'exemple 1, dans le cas où le nombre d'étages théoriques NET de contre-lavage est égal à 1. Le rendement d'extraction est de 99,1% pour le 5-HMF et de 8,7% pour le DMSO. Compte tenu du la composition de la charge, on obtient donc un extrait composé (en poids) de 19% de 5-HMF, 2% de DMSO et 79% de DCM.

On réalise alors une première étape d'évaporation d'une partie du solvant DCM contenu dans l'extrait, ce qui permet de concentrer le 5-HMF et le DMSO jusqu'à des concentrations de 30% et 3% respectivement (rapport pondéral DMSO/5-HMF de 0,1).

Cet extrait concentré est ensuite refroidi à une température de -18°C. Le 5-HMF cristallise alors avec un rendement de 22%. Le DMSO ne cristallise pas dans ces conditions et reste en solution.

Après une étape de filtration, on récupère ainsi un filtrat dont la composition pondérale est la suivante : 72,6% de DCM, 3,1% de DMSO et 24,3% de 5-HMF.

### Exemple 3 : Couplage du procédé selon l'invention avant NET=6 et du procédé de cristallisation à -18°C

Reprenons les résultats de l'exemple 1, dans le cas au le NET de contre-lavage est égal à 6. Le rendement d'extraction de 99% pour le 5-HMF et de 0% pour le DMSO. Compte tenu du la composition pondérale de la charge, on obtient donc un extrait composé (en poids) de 19% de 5-HMF et 81% de DCM.

On réalise alors une première étape d'évaporation d'une partie du solvant DCM contenu dans l'extrait, ce qui permet de concentrer le 5-HMF jusqu'à une concentration de 30% (rapport pondéral DMSO/5-HMF de 0).

Cet extrait concentré est ensuite refroidi à une température de -18°C. Le 5-HMF cristallise alors avec un rendement de 38,5% (cf. exemple 1).

Après une étape de filtration, on récupère ainsi un filtrat dont la composition pondérale est la suivante : 79,1% de DCM et 20,9%de 5-HMF.

### Exemple 4 : Couplage du procédé selon l'invention avant NET=6 et du procédé de cristallisation à -30°C

Reprenons les résultats de l'exemple 1, dans le cas au le NET de contre-lavage est égal à 6. Le rendement d'extraction de 99% pour le 5-HMF et de 0% pour le DMSO. Compte tenu du la composition pondérale de la charge, on obtient donc un extrait composé de (en poids) 19% de 5-HMF et 81% de DCM.

On réalise alors une première étape d'évaporation d'une partie du solvant DCM contenu dans l'extrait, ce qui permet de concentrer le 5-HMF jusqu'à une concentration de 30% (rapport pondéral DMSO/5-HMF de 0).

Cet extrait concentré est ensuite refroidi à une température de -30°C. Le 5-HMF cristallise alors avec un rendement de 84%.

Après une étape de filtration, on récupère ainsi un filtrat dont la composition est la suivante : 95,3% de DCM et 4,7%de 5-HMF.

En conclusion, ces différents cas d'étude montrent l'intérêt du procédé selon l'invention comportant une étape de contre-extraction qui permet de produire un extrait contenant le 5-HMF mais contenant peu ou pas de DMSO, ce qui facilite la cristallisation du 5-HMF.

### Exemple 5 : Comparaison avec l'art antérieur FR-2669635 en ce qui concerne la cristallisation du 5-HMF

On compare ici la cristallisation de deux extraits :
1) l'un contenant 30%pds de 5-HMF, 3%pds de DMSO et 67%pds de DCM, qui est produit dans les exemples du brevet FR 2669635,
2) l'autre contenant 30%pds de 5-HMF et 70%pds de DCM, qui est produit par la présente invention.

Ces deux extraits sont refroidis à une température de -18°C, et l'on observe la cristallisation du 5-HMF (F=30°C environ). En abaissant la température à composition constante, la concentration du 5-HMF dans le milieu devient supérieure à la limite de solubilité : on observe alors la nucléation de cristaux de 5-HMF et leur croissance, jusqu'à ce que la concentration de 5-HMF soit égale à la limite de solubilité à la température de -18°C. On note le temps τ nécessaire pour atteindre cet équilibre, et l'on détermine alors le rendement de cristallisation (quantité de 5-HMF cristallisé divisé par la quantité de 5-HMF initialement présent).

| Extrait | 1) Rapport pondéral DMSO/5-HMF = 0,1 | 2) Rapport pondéral DMSO/5-HMF = 0 |
|---|---|---|
| Rendement de cristallisation du 5-HMF | 22% | 38,5% |
| τ (jour) | 2 | <1 |

D'après les exemples fournis dans le brevet FR2669635, le rapport pondéral entre le DMSO et le 5-HMF dans les extraits est typiquement de l'ordre de 0,1. Ce rapport dépend bien entendu de nombreux facteurs, comme le rapport pondéral entre le DMSO et le 5-HMF dans la charge, la quantité d'eau ajouté à la charge (modifiant les coefficients de partage et donc la sélectivité d'extraction), ou bien encore le débit de solvant (i.e. rendement d'extraction). Néanmoins, le rapport pondéral DMSO/5-HMF ne peut être réduit à zéro dans l'invention décrite dans le brevet FR2669635.

Lorsque l'on cherche à cristalliser un extrait dans lequel le rapport pondéral de DMSO/5-HMF est de 0,1, comme dans les exemples fournis dans le brevet FR2669635, on constate que la nucléation et la croissance des cristaux est lente. De plus, à une température de -18°C, le rendement de cristallisation thermodynamiquement atteignable n'est que de 22%.

Lorsque la même expérience est réalisée avec un extrait ne contenant plus de DMSO, mais uniquement du 5-HMF, on observe une nucléation et croissance des cristaux à minima deux fois plus rapide, et le rendement de cristallisation thermodynamiquement atteignable est nettement augmenté, passant de 22% en présence de DMSO à 38,5% en absence de DMSO.

On comprend ici bien l'intérêt de produire un extrait ne contenant pas de DMSO, mais uniquement du 5-HMF, de manière à réduire les coûts d'opération et d'investissement associés à l'étape de cristallisation du 5-HMF.

## Revendications

1. Procédé de séparation du ou des composés furaniques contenus dans une charge comprenant également du diméthoxysulfoxyde (DMSO), ledit procédé comprenant successivement les étapes suivantes :
a) une étape a) dans laquelle ladite charge est mise en contact avec le raffinat intermédiaire aqueux enrichi en DMSO produit à l'étape c) et le mélange est éventuellement filtré,
b) une étape b) d'extraction dudit mélange produit à l'étape a) par un solvant organique choisi parmi les solvants chlorés, les éthers, les cétones et les aromatiques, produisant un raffinat aqueux enrichi en DMSO et un extrait organique intermédiaire enrichi en composé(s) furanique(s),
c) une étape c) de contre-extraction dudit extrait organique intermédiaire enrichi en composé(s) furanique(s) produit à l'étape b) par de l'eau ajoutée, produisant un raffinat intermédiaire aqueux enrichi en DMSO et un extrait organique purifié, et ledit raffinat intermédiaire aqueux est introduit en partie ou en totalité dans l'étape a).

2. Procédé selon la revendication 1 dans lequel le composé furanique est choisi dans le groupe formé par le 5-hydroxyméthylfurfural (5-HMF), le 2,5-diformylfurane (DFF), l'acide 2,5-furanedicarboxylique (FDCA) ou le 2,5-dimethyl furanoate dicarboxylique (DMFDCA), seuls ou en mélange.

3. Procédé selon l'une des revendications précédentes dans lequel le composé furanique est le 5-hydroxyméthylfurfural (5-HMF).

4. Procédé selon l'une des revendications précédentes comportant en outre une étape d) de cristallisation du composé furanique, en particulier du 5-HMF, suivie d'une filtration et il est obtenu ledit composé solide d'une part et un solvant riche en solvant d'autre part.

5. Procédé selon l'une des revendications précédentes dans lequel le raffinat aqueux enrichi en DMSO produit à l'étape b) est distillé, de préférence sous vide, il est obtenu un résidu riche en DMSO d'une part et un distillat riche en eau d'autre part.

6. Procédé selon la revendication 5 dans lequel le distillat riche en eau est recyclé, en partie ou en totalité, à l'étape c).

7. Procédé selon l'une des revendications précédentes dans lequel le solvant de l'étape b) est choisi dans le groupe formé par les solvants chlorés C1-C10, les éthers C2-C10, les cétones C2-C10, des composés aromatiques C4-C10.

8. Procédé selon l'une des revendications précédentes dans lequel le solvant est choisi dans le groupe formé par le dichlorométhane, le diethyléther, le diisopropyléther, la méthyléthylcétone, la methylisopropylcétone, le thiophène, l'anisole et le toluène, et de préférence le solvant est le dichlorométhane.

9. Procédé selon l'une des revendications précédentes dans lequel les étapes b) et c) sont réalisées à contre-courant à une température comprise entre 0°C et 60°C, de préférence entre 10°C et 30°C.

10. Procédé selon l'une des revendications précédentes dans lequel l'eau de contre-extraction introduite à l'étape c) contient moins de 1%pds de DMSO.

11. Procédé selon l'une des revendications précédentes dans lequel le filtrat issu de l'étape d) est recyclé, en partie ou en totalité, vers l'étape b).

12. Procédé selon l'une des revendications précédentes dans lequel la quantité d'eau dans ledit mélange étant de10% à 90% pds.

13. Procédé selon l'une des revendications précédentes dans lequel à l'étape c), ledit raffinat aqueux intermédiaire contient au moins 60% pds d'eau.

14. Procédé selon l'une des revendications précédentes dans lequel les étapes b) et c) sont réalisées dans des zones différentes séparées.

15. Procédé selon l'une des revendications précédentes dans lequel la quantité d'humines dans la charge étant de 1% poids ou moins, les étapes a), b) et c) sont réalisées dans des zones différentes non séparées.

## Patentansprüche

1. Verfahren zur Abtrennung der Furanverbindung bzw. der Furanverbindungen, die in einem Einsatzstoff enthalten sind, der auch Dimethoxysulfoxid (DMSO) enthält, wobei das Verfahren nacheinander die folgenden Schritte umfasst:
a) einen Schritt a), in dem der Einsatzstoff mit dem in Schritt c) erhaltenen mit DMSO angereicherten wässrigen intermediären Raffinat in Kontakt gebracht und die Mischung gegebenenfalls filtriert wird,
b) einen Schritt b) der Extraktion der in Schritt a) erhaltenen Mischung mit einem aus chlorierten Lösungsmitteln, Ethern, Ketonen und Aromaten ausgewählten organischen Lösungsmittel unter Erhalt eines mit DMSO angereicherten wässrigen Raffinats und eines mit Furanverbindung (en) angereicherten intermediären organischen Extrakts,
c) einen Schritt c) der Rückextraktion des in Schritt b) erhaltenen mit Furanverbindung(en) angereicherten intermediären organischen Extrakts mit zugesetztem Wasser unter Erhalt eines mit DMSO angereicherten wässrigen intermediären Raffinats und eines gereinigten organischen Extrakts, wobei das wässrige intermediäre Raffinat teilweise oder ganz in Schritt a) eingetragen wird.

2. Verfahren nach Anspruch 1, wobei die Furanverbindung aus der Gruppe bestehend aus 5-Hydroxymethylfurfural (5-HMF), 2,5-Diformylfuran (DFF), 2,5-Furandicarbonsäure (FDCA) und 2,5-Furandicarbonsäuredimethylester (DMFDCA) alleine oder als Gemisch ausgewählt wird.

3. Verfahren nach einem der vorhergehenden Ansprüche, wobei es sich bei der Furanverbindung um 5-Hydroxymethylfurfural (5-HMF) handelt.

4. Verfahren nach einem der vorhergehenden Ansprüche, das außerdem einen Schritt d) der Kristallisation der Furanverbindung, insbesondere von 5-HMF, mit anschließender Filtration umfasst und einerseits die feste Verbindung und andererseits ein lösungsmittelreiches Lösungsmittel erhalten wird.

5. Verfahren nach einem der vorhergehenden Ansprüche, wobei das in Schritt b) erhaltene mit DMSO angereicherte wässrige Raffinat destilliert wird, vorzugsweise unter Vakuum, einerseits ein DMSOreicher Rückstand und andererseits ein wasserreiches Destillat erhalten wird.

6. Verfahren nach Anspruch 5, wobei das wasserreiche Destillat teilweise oder ganz in Schritt c) zurückgeführt wird.

7. Verfahren nach einem der vorhergehenden Ansprüche, wobei das Lösungsmittel von Schritt b) aus der Gruppe bestehend aus chlorierten C1-C10-Lösungsmitteln, C2-C10-Ethern, C2-C10-Ketonen und aromatischen C4-C10-Verbindungen ausgewählt wird.

8. Verfahren nach einem der vorhergehenden Ansprüche, wobei das Lösungsmittel aus der Gruppe bestehend aus Dichlormethan, Diethylether, Diisopropylether, Methylethylketon, Methylisopropylketon, Thiophen, Anisol und Toluol ausgewählt wird und es sich vorzugsweise bei dem Lösungsmittel um Dichlormethan handelt.

9. Verfahren nach einem der vorhergehenden Ansprüche, wobei die Schritte b) und c) im Gegenstrom bei einer Temperatur zwischen 0 °C und 60 °C, vorzugsweise zwischen 10 °C und 30 °C, durchgeführt werden.

10. Verfahren nach einem der vorhergehenden Ansprüche, wobei das in Schritt c) eingetragene Wasser für die Rückextraktion weniger als 1 Gew.-% DMSO enthält.

11. Verfahren nach einem der vorhergehenden Ansprüche, wobei das Filtrat aus Schritt d) teilweise oder ganz in Schritt b) zurückgeführt wird.

12. Verfahren nach einem der vorhergehenden Ansprüche, wobei die Wassermenge in der Mischung 10 bis 90 Gew.-% beträgt.

13. Verfahren nach einem der vorhergehenden Ansprüche, wobei in Schritt c) das intermediäre wässrige Raffinat mindestens 60 Gew.-% Wasser enthält.

14. Verfahren nach einem der vorhergehenden Ansprüche, wobei die Schritte b) und c) in getrennten verschiedenen Zonen durchgeführt werden.

15. Verfahren nach einem der vorhergehenden Ansprüche, wobei die Menge an Huminen im Einsatzstoff 1 Gew.-% oder weniger beträgt und die Schritte a), b) und c) in nicht getrennten verschiedenen Zonen durchgeführt werden.

## Claims

1. Process for the separation of the furan compound(s) contained in a feedstock also comprising dimethoxysulfoxide (DMSO), said process successively comprising the following stages:
a) a stage a) in which said feedstock is brought into contact with the intermediate aqueous raffinate enriched in DMSO produced in stage c) and the mixture is optionally filtered,
b) a stage b) of extraction of said mixture produced in stage a) by an organic solvent chosen from chlorinated solvents, ethers, ketones and aromatic compounds, producing an aqueous raffinate enriched in DMSO and an intermediate organic extract enriched in furan compound(s),
c) a stage c) of backextraction of said intermediate organic extract enriched in furan compound(s) produced in stage b) by water added, producing an intermediate aqueous raffinate enriched in DMSO and a purified organic extract, and said intermediate aqueous raffinate is introduced, partially or completely, in stage a).

2. Process according to Claim 1, in which the furan compound is chosen from the group formed by 5-hydroxymethylfurfural (5-HMF), 2,5-diformylfuran (DFF), 2,5-furandicarboxylic acid (FDCA) and dimethyl 2,5-furandicarboxylate (DMFDCA), alone or as a mixture.

3. Process according to either of the preceding claims, in which the furan compound is 5-hydroxymethylfurfural (5-HMF).

4. Process according to one of the preceding claims, additionally comprising a stage d) of crystallization of the furan compound, in particular 5-HMF, followed by a filtration, and there is obtained said solid compound, on the one hand, and a solvent rich in solvent, on the other hand.

5. Process according to one of the preceding claims, in which the aqueous raffinate enriched in DMSO produced in stage b) is distilled, preferably under vacuum, a residue rich in DMSO, on the one hand, and a distillate rich in water, on the other hand, are obtained.

6. Process according to Claim 5, in which the distillate rich in water is recycled, partially or completely, in stage c).

7. Process according to one of the preceding claims, in which the solvent of stage b) is chosen from the group formed by C₁-C₁₀ chlorinated solvents, C₂-C₁₀ ethers, C₂-C₁₀ ketones and C₄-C₁₀ aromatic compounds.

8. Process according to one of the preceding claims, in which the solvent is chosen from the group formed by dichloromethane, diethyl ether, diisopropyl ether, methyl ethyl ketone, methyl isopropyl ketone, thiophene, anisole and toluene, and preferably the solvent is dichloromethane.

9. Process according to one of the preceding claims, in which stages b) and c) are carried out countercurrentwise at a temperature of between 0°C and 60°C, preferably between 10°C and 30°C.

10. Process according to one of the preceding claims, in which the backextraction water introduced in stage c) contains less than 1% by weight of DMSO.

11. Process according to one of the preceding claims, in which the filtrate resulting from stage d) is recycled, partially or completely, to stage b).

12. Process according to one of the preceding claims, in which the amount of water in said mixture is from 10% to 90% by weight.

13. Process according to one of the preceding claims, in which, in stage c), said intermediate aqueous raffinate contains at least 60% by weight of water.

14. Process according to one of the preceding claims, in which stages b) and c) are carried out in different separate zones.

15. Process according to one of the preceding claims, in which the amount of humins in the feedstock being 1% by weight or less, stages a), b) and c) are carried out in different non-separated zones.
